(19) Europäisches Patentamt / European Patent Office / Office européen des brevets

(11) **EP 4 349 823 A1**

(12) **EUROPEAN PATENT APPLICATION**
published in accordance with Art. 153(4) EPC


(43) Date of publication:
**10.04.2024 Bulletin 2024/15**

(21) Application number: **22815792.1**

(22) Date of filing: **10.05.2022**

(51) International Patent Classification (IPC):
***C07D 319/06** (2006.01)* ***C08G 59/50** (2006.01)*
***C07B 61/00** (2006.01)* ***C08L 63/00** (2006.01)*
***C07D 493/10** (2006.01)*

(52) Cooperative Patent Classification (CPC):
**C07B 61/00; C07D 319/06; C07D 493/10; C08G 59/50; C08L 63/00**

(86) International application number:
**PCT/JP2022/019776**

(87) International publication number:
**WO 2022/255037 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.05.2021 JP 2021091643**



(71) Applicant: **MITSUBISHI GAS CHEMICAL COMPANY, INC.**
**Chiyoda-ku**
**Tokyo 100-8324 (JP)**

(72) Inventors:
- **YOKOO YAMAZOE, Aoi**
  **Tokyo 100-8324 (JP)**
- **KANBARA, Yutaka**
  **Niigata-shi, Niigata 950-3112 (JP)**
- **KOUNO, Kazuki**
  **Hiratsuka-shi, Kanagawa 254-0016 (JP)**
- **OHNO, Yuma**
  **Hiratsuka-shi, Kanagawa 254-0016 (JP)**

(74) Representative: **Hoffmann Eitle**
**Patent- und Rechtsanwälte PartmbB**
**Arabellastraße 30**
**81925 München (DE)**


# (54) NOVEL AMINO COMPOUND AND METHOD FOR PRODUCING SAME, AND EPOXY RESIN CURING AGENT, EPOXY REIN COMPOSITION AND EPOXY RESIN CURED PRODUCT USING SAME


(57) For providing a novel amino compound useful for an epoxy resin curing agent and a method for producing the same, the amino compound according to the present disclosure has a quaternary carbon at the β-position of an amino group and is represented by the following formula (1).

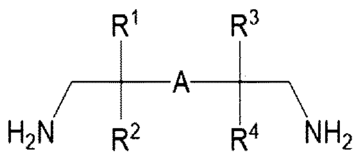

···(1)

wherein,
A is any of;
(α) a hydrocarbon group independently having one or more -O- bond bonded to the carbon at the γ-position of the amino group, and having 2 to 30 carbon atoms and optionally having a substituent (the same hereinafter),
(β) a hydrocarbon group having one or more -O-bond bonded to the carbon at the γ-position of the amino group while bonding to any one of $R^1$ to $R^4$, and having 2 to 30 carbon atoms, and
(γ) a hydrocarbon group having one or more -O-bond bonded to the carbon at the γ-position of the amino group while bonding to $R^1$ or $R^2$ and $R^3$ or $R^4$, and having 2 to 30 carbon atoms, wherein

$R^1$ to $R^4$ are each independently a hydrocarbon group having 1 to 10 carbon atoms (provided that a hydrocarbon group bonded to A is excluded).


EP 4 349 823 A1

## Description

Technical Field

The present invention provides an amino compound, which is a novel compound, and a method for producing the same, and also relates to an epoxy resin curing agent, an epoxy resin composition, and an epoxy resin cured product containing a novel amino compound.

Background Art

Amino compounds that have been conventionally used as epoxy resin curing agents include, for example, diethylenetriamine, triethylenetetramine, polyoxypropylenediamine (registered trademark: Jeffamine), isophoronediamine (IPDA), metaxylylenediamine, 1,3-bis(aminomethyl)cyclohexane (1,3-BAC), and the like. These are industrially produced, and epoxy resin curing agents containing an amino compound have been sold. Additionally, general reviews of performances of various amines have been published (for example, see Non Patent Literature 1 below).

Citation List

Patent Literature

Non Patent Literature 1:
Sosetsu Epoxy Resin (in Japanese) (General review of epoxy resins) (The Japan Society of Epoxy Resin Technology)

Summary of Invention

Technical Problem

Generally, an amino compound having a primary amino group reacts fast to an epoxy resin and has fast curing property. On the other hand, the time that takes to gelling due to since blended is short, thereby posing a drawback of limiting work time once blended. In particular, depending on the purpose of use of an epoxy resin composition, fast curing is not suitable for use. For example, one of the methods for producing a fiberreinforced composite material (hereinafter, also referred to as "FRP (Fiber Reinforced Plastics)") includes the filament winding method. The filament winding method is a method of coating the outer surface of a mandrel, and the like, using reinforced fiber yarns with which a matrix resin or a precursor thereof is impregnated, and forming a formed article. A thermosetting resin composition such as an epoxy resin composition can be used as a matrix resin precursor, but when such a resin composition has a short pot life and is fast curing, a problem arises that the thermosetting resin cures at a pre-formation stage.

Under the circumstances, the present invention has been completed in view of the above problems, and aims to provide a novel amino compound that was not known so far to be useful as an epoxy resin curing agent and a method for producing the same, and an epoxy resin curing agent, an epoxy resin composition, and an epoxy resin cured product using the same.

Solution to Problem

The present inventors have conducted extensive studies to solve the above problem and found that the use of an amino compound having a specific structure prevents the excessive fast curing, which is the problem described above, and can achieve a long pot life thereby enhancing the workability, whereby the present invention has been accomplished. That is, the contents of the present disclosure are as follows.

[1] An amino compound having a quaternary carbon at the β-position of an amino group and represented by the following formula (1).

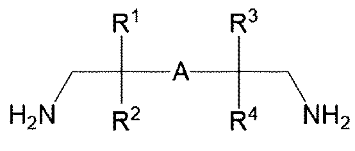

···(1)

wherein,

A is any of the following (α), (β), and (γ);

(α) a hydrocarbon group independently having at least one -O- bond bonded to the carbon at the γ-position of the amino group, and having 2 to 30 carbon atoms and optionally having a substituent,
(β) a hydrocarbon group having at least one -O-bond bonded to the carbon at the γ-position of the amino group while bonding to any one of $R^1$, $R^2$, $R^3$, and $R^4$, and having 2 to 30 carbon atoms and optionally having a substituent,
(γ) a hydrocarbon group having at least one -O-bond bonded to the carbon at the γ-position of the amino group while bonding to $R^1$ or $R^2$ and $R^3$ or $R^4$, and having 2 to 30 carbon atoms and optionally having a substituent, wherein

$R^1$, $R^2$, $R^3$, and $R^4$ are each independently a hydrocarbon group having 1 to 10 carbon atoms and optionally having a substituent (provided that a hydrocarbon group bonded to A is excluded).

[2] The amino compound according to the above [1],

wherein A independently has a dioxane structure, or
A has a dioxane structure while bonding to any one of $R^1$, $R^2$, $R^3$, and $R^4$, or
A has a dioxane structure while bonding to $R^1$ or $R^2$ and $R^3$ or $R^4$.

[3] The amino compound according to the above [1] or [2], represented by the following formula (2) or formula (3).

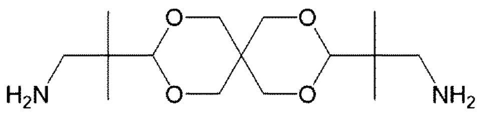 ···(2) 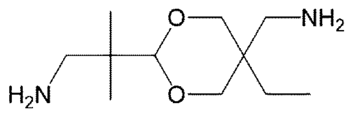 ···(3)

[4] A method for producing an amino compound comprising:
a step of aminizing a polyol compound represented by the following formula (1a) in the presence of a catalyst and ammonia to obtain an amino compound having a quaternary carbon at the β-position of an amino group and represented by the following formula (1).

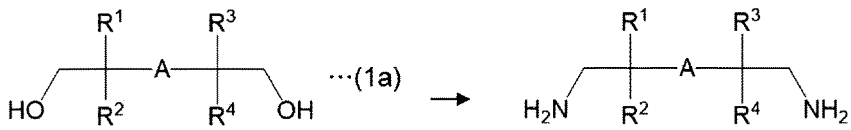
···(1a) ···(1)

In formula (1) and formula (1a),

A is any of the following (α), (β), and (γ);

(α) a hydrocarbon group independently having at least one -O- bond bonded to the carbon at the γ-position of the amino group, and having 2 to 30 carbon atoms and optionally having a substituent,
(β) a hydrocarbon group having at least one -O-bond bonded to the carbon at the γ-position of the amino group while bonding to any one of $R^1$, $R^2$, $R^3$, and $R^4$, and having 2 to 30 carbon atoms and optionally having a substituent,
(γ) a hydrocarbon group having at least one -O-bond bonded to the carbon at the γ-position of the amino group while bonding to $R^1$ or $R^2$ and $R^3$ or $R^4$, and having 2 to 30 carbon atoms and optionally having a substituent, wherein

$R^1$, $R^2$, $R^3$, and $R^4$ are each independently a hydrocarbon group having 1 to 10 carbon atoms and optionally having a substituent (provided that a hydrocarbon group bonded to A is excluded).

[5] The method for producing the amino compound according to the above [4],

wherein A independently has a dioxane structure, or
A has a dioxane structure while bonding to any one of $R^1$, $R^2$, $R^3$, and $R^4$.

[6] A method for producing an amino compound comprising:

a step of aminizing a polyol compound represented by the following formula (2a) in the presence of a catalyst and ammonia to obtain an amino compound represented by the following formula (2), or
a step of aminizing a polyol compound represented by the following formula (3a) in the presence of a catalyst and ammonia to obtain an amino compound represented by the following formula (3).

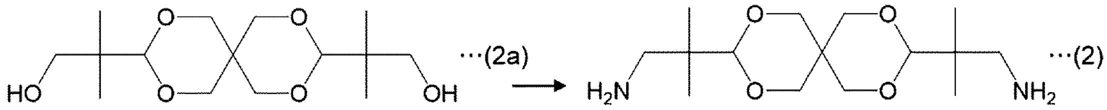
···(2a) ···(2)

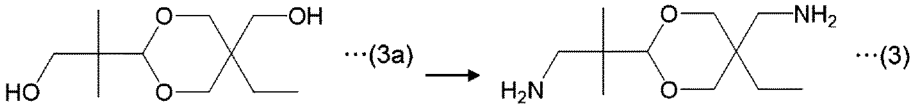
···(3a) ···(3)

[7] An epoxy resin curing agent, comprising the amino compound according to any one of the above [1] to [3].
[8] An epoxy resin composition comprising the epoxy resin curing agent according to the above [7], and an epoxy resin.
[9] An epoxy resin cured product obtained by curing the epoxy resin composition according to the above [8].

Advantageous Effects of Invention

The present disclosure can accordingly provide a novel amino compound that is useful to be an epoxy resin curing agent, which excels particularly in a long pot life, water spot resistance, and chemical resistance while having a good coating film appearance and coating film drying property, a method for producing the same, and an epoxy resin curing agent, an epoxy resin composition, and an epoxy resin cured product using the same.

Description of Embodiments

Hereinafter, embodiments of the present disclosure will be described. The following embodiments are examples for describing the present disclosure, and the present disclosure is not limited only to these embodiments, and various modifications can be made without departing from the gist thereof.

[Amino compound]

The amino compound according to the present embodiment is a compound having a quaternary carbon at the β-position of an amino group, and at least one -O-bond bonded to the carbon at the γ-position of the amino group (in other words, a moiety in which -O- bonds to the carbon at the γ-position of the amino group), and is specifically represented by the following formula (1).

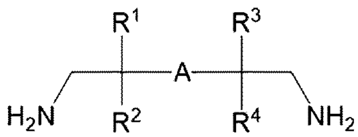
···(1)

wherein, A is any of the following (α), (β), and (γ). That is, A is

(α) a hydrocarbon group independently having at least one -O- bond bonded to the carbon at the γ-position of the amino group, and having 2 to 30 carbon atoms and optionally having a substituent, or
(β) a hydrocarbon group having at least one -O- bond
bonded to the carbon at the γ-position of the amino group while bonding to any one of $R^1$, $R^2$, $R^3$, and $R^4$, and having 2 to 30 carbon atoms and optionally having a substituent, or
(γ) a hydrocarbon group having at least one -O- bond
bonded to the carbon at the γ-position of the amino group while bonding to $R^1$ or $R^2$ and $R^3$ or $R^4$, and having 2 to 30 carbon atoms and optionally having a substituent.

As described, A is a hydrocarbon group containing an oxygen atom as the heteroatom.

Additionally, in formula (1), $R^1$, $R^2$, $R^3$, and $R^4$ are each independently a hydrocarbon group having 1 to 10 carbon atoms and optionally having a substituent. However, $R^1$, $R^2$, $R^3$, and/or $R^4$ bonding to A, as in (β) and (γ) described above, forms a part of A, for the reason of which these are excluded from the definition herein.

Examples of the hydrocarbon group having 1 to 30 carbon atoms at $R^1$, $R^2$, $R^3$, and $R^4$ include alkyl groups and alkoxy groups having 1 to 10 carbon atoms, such as methyl group, and ethyl group; straight or branched-chain propyl group, butyl group, pentyl group, hexyl group, heptyl group, octyl group, nonyl group, and decyl group; and cyclic cyclohexyl group, cyclopentyl group, phenyl group, benzyl group, bicyclic hydrocarbon group having a spiro-cyclic skeleton. Additionally, examples of the substituent at A, and $R^1$, $R^2$, $R^3$, and $R^4$ include hydroxy group and carbonyl group.

More specifically, it is preferable that A independently has a dioxane structure, or has a dioxane structure while bonding to any one of $R^1$, $R^2$, $R^3$, and $R^4$. In this case, more preferable examples of the amino compound of formula (1) in the present embodiment include, further specifically, 2,2'-(2,4,8,10-tetraoxaspiro[5.5]undecane-3,9-diyl)bis(2-methylpropane-1-amine) represented by the following formula (2), and 2-(5-(aminomethyl)-5-ethyl-1,3-dioxan-2-yl)-2-methylpropane-1-amine represented by the following formula (3).

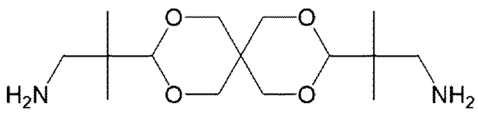 ···(2)

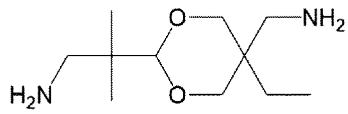 ···(3)

Additionally, other examples of the amino compound represented by the above formula (1) include 3-amino-2,2-dimethylpropyl 3-amino-2,2-dimethylpropanoate represented by the following formula (4), 2,2'-(oxybis(methylene))bis(2-(aminomethyl)butan-1-ol) represented by the following formula (5-1), 2-((2,2-bis(aminomethyl)butoxy)methyl-2-ethylpropane-1,3-diol represented by the following formula (5-2), 2-(aminomethyl)-2-((2,2-bis(aminomethyl)butoxy)methyl)butan-1-ol) represented by the following formula (5-3), 2,2'-(oxybis(methylene))bis(2-ethylpropane-1,3-diamine) represented by the following formula (5-4), 3,3'-oxybis(2,2-dimethylpropane-1-amine) represented by the following formula (6), 2-((3-amino-2,2-dimethylpropoxy)methyl)-2-ethylhexane-1-amine represented by the following formula (7), 2-((3-amino-2,2-dimethylpropoxy)methyl)-2-(aminomethyl)butan-1-ol represented by the following formula (8-1), 3-(2,2-bis(aminomethyl)butoxy)-2,2-dimethylpropan-1-ol represented by the following formula (8-2), and 2-((3-amino-2,2-dimethylpropoxy)methyl)-2-ethylpropane-1,3-diamine represented by the following formula (8-3).

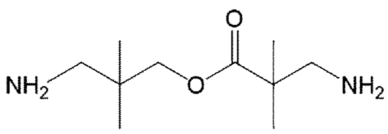 ···(4)

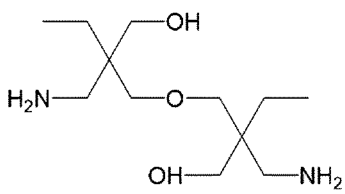 ···(5-1)

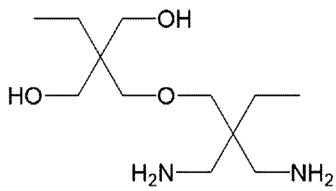 ···(5-2)

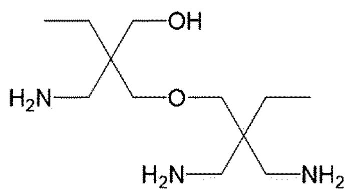 ···(5-3)

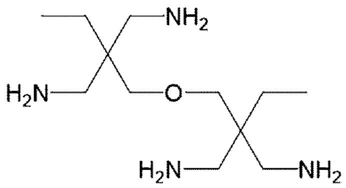 ···(5-4)

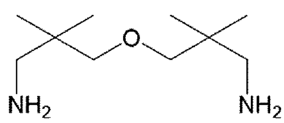 ···(6)

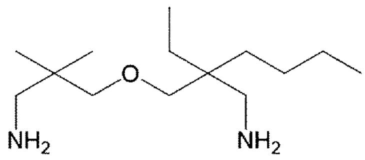 ···(7)

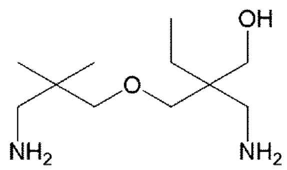 ···(8-1)

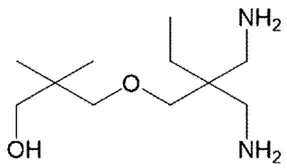 ···(8-2)

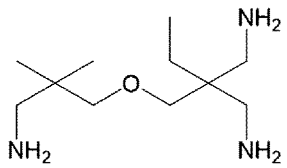 ···(8-3)

Further, other examples of the amino compound represented by the above formula (1) include (3,12-diethyl-1,5,10,14-tetraoxadispiro[5.2.5$^{9}$.2$^{6}$]hexadecane-3,12-diyl)dimethaneamine represented by the following formula (9), and the compounds represented by the following formula (10). In formula (10), X is a group selected from the group consisting of straight-chain alkyl groups having 0 to 6 carbon atoms, branched-chain alkyl groups and aryl groups having 3 to 6 carbon atoms, and groups having 6 to 20 carbon atoms. Examples of such compound represented by the formula (10) include (propane-2,2-diylbis(3-ethyl-1,5-dioxaspiro[5.5]undecane-9.3-diyl))dimethanamine represented by the following formula (10-1), and ((9H-fluorene-9,9-diyl)bis(3-ethyl-1,5-dioxaspiro[5,5]undecane-9,3-diyl)dimethanamine represented by the following formula (10-2).

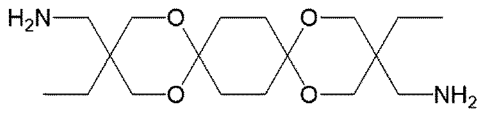 ···(9)

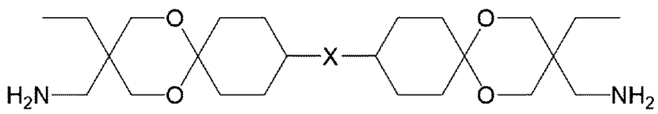 ···(10)

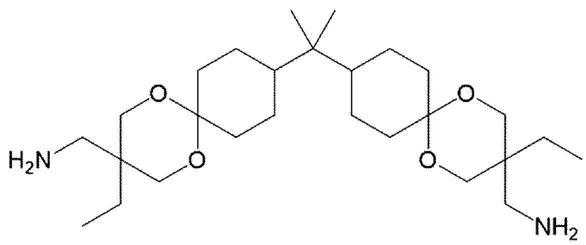 ···(10-1)

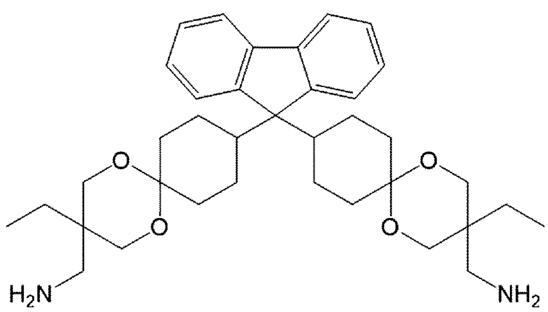 ···(10-2)

As described, the compound (spiro bisamine) represented by formula (2), the compound represented by formula (4), the compounds represented by formula (5-1) to formula (5-4), the compound represented by formula (6), the compound represented by formula (7), and the compounds represented by formula (8-1) to formula (8-3) are considered as examples of the amino compound containing A of (α) described above. Further, the compound (dioxane bisamine) represented by formula (3) is considered as an example of the amino compound containing A of (β) described above.

Further, the compound represented by formula (9), the compound represented by formula (10), the compound represented by formula (10-1), and the compound represented by formula (10-2) are considered as examples of the amino compound containing A of (γ) described above.

[Method for producing amino compounds]

The production method of the amino compound represented by the above formula (1) is not particularly limited but, for example, as described below, the amino compound can be obtained by aminizing the polyol compound represented by formula (1a).

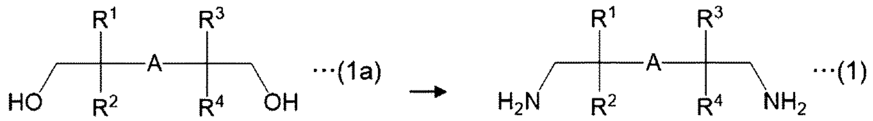

In formula (1) and formula (1a), A is, as is mentioned above, any of the following (α), (β), and (γ). That is, A is

(α) a hydrocarbon group independently having at least one -O- bond bonded to the carbon at the γ-position of the amino group, and having 2 to 30 carbon atoms and optionally having a substituent, or
(β) a hydrocarbon group having at least one -O- bond bonded to the carbon at the γ-position of the amino group while bonding to any one of $R^1$, $R^2$, $R^3$, and $R^4$, and having 2 to 30 carbon atoms and optionally having a substituent, or
(γ) a hydrocarbon group having at least one -O- bond bonded to the carbon at the γ-position of the amino group while bonding to $R^1$ or $R^2$ and $R^3$ or $R^4$, and having 2 to 30 carbon atoms and optionally having a substituent.

The amination reaction herein is not particularly limited but, for example, it is preferable that a polyol compound, which is a starting material, and ammonia are reacted in the presence of a reduction catalyst. For the reduction catalyst, any catalyst typically used for the amination reaction can be used. For example, usable catalysts include precious metal catalysts such as platinum, palladium, ruthenium, and rhodium; and metalbased catalysts such as nickel, cobalt, and iron catalyst. These catalysts can be used as metal independently, or used as supported on a carrier such as activated carbon, alumina, silica, titania, and zirconia. The catalysts can be used singly, or two or more can be used in combination. Of these, it is further preferable to use a catalyst supporting ruthenium from the viewpoint of increasing the yield of the amino compound.
The mass ratio of the polyol compound to the reduction catalyst is preferably 2 to 30, more preferably 5 to 20, and further preferably 5 to 15. When a mass ratio of the polyol compound to the reduction catalyst is within the above ranges, the yield and selectivity of the amino compound to be obtained are likely to increase.
The molar ratio of ammonia to the polyol compound is preferably 2 to 150, more preferably 5 to 100, and further preferably 8 to 80. When a molar ratio is a lower limit value or more, the reaction is likely to be fast, whereas when a molar ratio is an upper limit value or less, the subsequent time to remove liquid ammonia is reduced, and the production efficiency improves. Ammonia can also be used as a solvent, and ammonia can be in any form and used in forms of, for example, liquid ammonia, and ammonia water.
The amination reaction is not particularly needed, but a solvent typically used for the amination reaction can be used. Examples include benzene, toluene, xylene, nitromethane, nitrobenzene, carbon disulfide, acetonitrile, benzonitrile, hexane, cyclohexane, petroleum ether, diethyl ether, 1,4-dioxane, methyl acetate, tetrahydrofuran, acetone, methyl ethyl ketone, dichloroethane, dimethylformamide, dimethyl sulfoxide, dimethyl carbonate, and propylene carbonate. These solvents can be used singly, or two or more can be used in combination.
The reaction temperature of the amination reaction is preferably 100 to 350°C, more preferably 150 to 300°C, and further preferably 200 to 250°C. When a reaction temperature of the amination reaction is a lower limit value or more, the reactivity improves, whereas a reaction temperature of the amination reaction is an upper limit value or less, high-boiling compounds are likely to be reduced.
Further, the initial hydrogen charging pressure (excluding the self pressure of liquid ammonia) of the amination reaction is preferably 0 to 20 MPa, more preferably 0.1 to 10 MPa, and further preferably 1 to 8 MPa. When a pressure of the amination reaction is within the above ranges, the yield and selectivity of the amino compound to be obtained are likely to increase.
Furthermore, the amination reaction is carried out until the reaction completes, but examples of the reaction time include 1 to 48 hours, 2 to 24 hours, and 4 to 10 hours.
More specifically, it is preferable that A in formula (1) and formula (1a) independently has a dioxane structure, or has a dioxane structure while bonding to any one of $R^1$, $R^2$, $R^3$, and $R^4$. In this case, more preferable examples of the method for producing the amino compound of formula (1) in the present embodiment include, further specifically, in

the presence of a suitable catalyst and ammonia, a method of aminizing 2,2'-(2,4,8,10-tetraoxaspiro[5.5]undecane-3,9-diyl)bis(2-methylpropan-1-ol) represented by the following formula (2a) described above to obtain the amino compound of formula (2), and a method of aminizing 2-(5-ethyl-5-(hydroxymethyl)-1,3-dioxan-2-yl)-2-methylpropan-1-ol represented by the following formula (3a) to obtain the amino compound of formula (3).

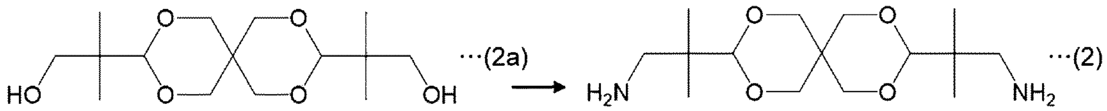

···(2a) ···(2)

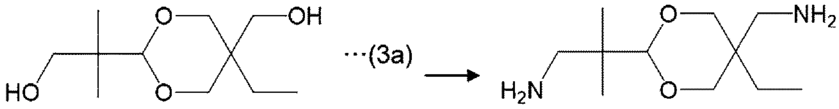

···(3a) ···(3)

Additionally, other examples of the polyol compound represented by the above formula (1a) include 3-hydroxy-2,2-dimethylpropyl 3-hydroxy-2,2-dimethylpropanoate represented by the following formula (4a), 2,2'-(oxybis(methylene))bis(2-ethylpropane-1,3-diol) represented by the following formula (5a), 3,3'-oxybis(2,2-dimethylpropan-1-ol) represented by the following formula (6a), 2-ethyl-2-((3-hydroxy-2,2-dimethylpropoxy)methyl)hexan-1-ol represented by the following formula (7a), and 2-ethyl-2-((3-hydroxy-2,2-dimethylpropoxy)methyl)propane-1,3-diol represented by the following formula (8a).

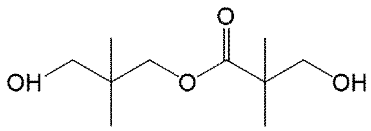

···(4a)

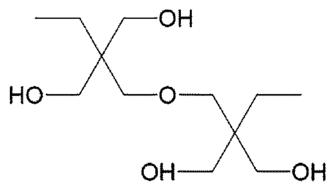

···(5a)

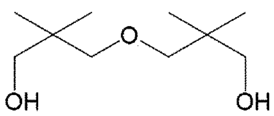

···(6a)

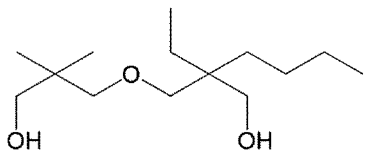

···(7a)

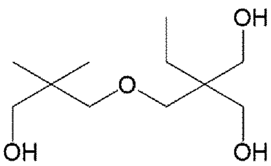

···(8a)

Further, other examples of the polyol compound represented by the above formula (1a) include (3,12-diethyl-1,5,10,14-tetraoxadispiro[5.2.5$^{9}$.2$^{6}$]hexadecane-3,12-diyl)dimethanol represented by the following formula (9a), compounds represented by the following formula (10a) (X is the same as in the above formula (10), (propane-2,2-diylbis(3-ethyl-1,5-dioxaspiro[5.5]undecane-9,3-diyl))dimethanol represented by the following formula (10a-1), and ((9H-fluorene-9,9-diyl)bis(3-ethyl-1,5-dioxaspiro[5.5]undecane-9,3-diyl))dimethanol represented by the following formula (10a-2).

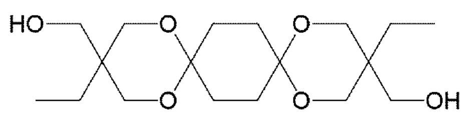

···(9a)

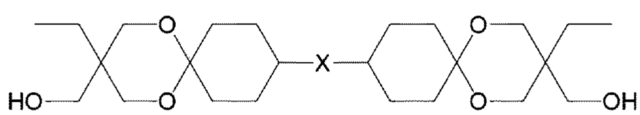

···(10a)

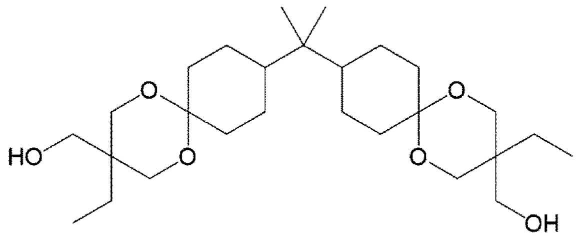

···(10a-1)

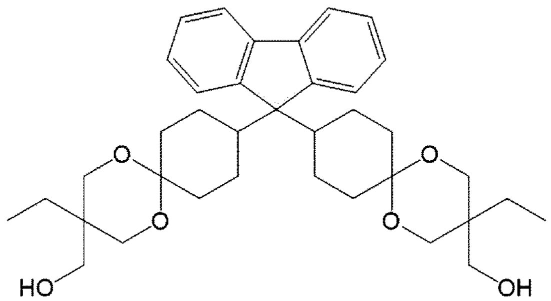

···(10a-2)

When such an amino compound according to the present disclosure is used as an epoxy resin curing agent, an epoxy resin composition and an epoxy resin cured product, as to be described later, that excel particularly in a longer pot life, water spot resistance, and chemical resistance compared with the conventionally used amino compounds while having a good coating film appearance and coating film drying property, can be obtained. Details of the action mechanism thereof are still unknown in part, but in the amino compounds according to the present disclosure, it is presumed that the quaternary carbon located at the β-position of an amino group, or, in addition thereto, the A moiety containing an -O- bond bonded to the carbon at the γ-position of the amino group becomes steric hinderance and blocks the curing reaction of an epoxy resin, thereby achieving outstanding excellent characteristics, particularly, in the viewpoint of the long pot life. However, the action is not limited thereto.

[Epoxy resin curing agent]

The amino compound represented by the above formula (1) can be suitably used as an epoxy resin curing agent. When the amino compound represented by formula (1) is used as an epoxy resin curing agent, other amine compounds contributing to the curing of an epoxy resin can also be used in combination. In such a case, it is preferable that an amount of the amino compound represented by formula (1) is 20 mass% or more to the total amount of the amino compound represented by formula (1) and other amine compounds. Further, for more benefiting the features of the epoxy resin curing agent, the amount of the amino compound represented by formula (1) to the total amount of the amino compound represented by formula (1) and other amine compounds is more preferably 30 mass% or more, and further preferably 50 mass% or more. Further, the epoxy resin curing agent according to the present disclosure that uses the amino compound represented by formula (1) may contain, in addition to other amine compounds described above, a component that does not contribute to the curing of a solvent, and the like, in the range in which the effects of the present invention are not affected. Furthermore, the epoxy resin curing agent according to the present disclosure, when used as an epoxy resin composition to be described later, can be obviously used in combination with other curing agents.

[Epoxy resin composition]

The epoxy resin composition according to the present disclosure contains an epoxy resin and the epoxy resin curing agent that uses the amino compound represented by the above formula (1). The epoxy resin used in the epoxy resin composition according to the present disclosure is not particularly limited as long as an epoxy resin has a glycidyl group reactable to the active hydrogen derived from the amino group contained in the epoxy resin curing agent according to the present disclosure and, for example, bisphenol A epoxy resin, bisphenol F epoxy resin, or an epoxy resin having a mixture of these as the main component can be suitably used. Additionally, the epoxy resin composition according to the present disclosure, in accordance with the purpose of use, can suitably use additives including modification components such as a filler, and a plasticizer, a reactive or unreactive diluent, flow regulating components such as a thixotropic agent, components such as a pigment, a leveling agent, and a tackifier, a cissing inhibitor, an anti-sagging agent, a flowing agent, a defoaming agent, an ultraviolet absorbent, and a light stabilizer. For the mixing ratio of the epoxy resin

and the epoxy resin curing agent in the epoxy resin composition according to the present disclosure, the ratio in the number of functional groups [ep] calculated from an epoxy equivalent weight of the epoxy resin to the number of functional groups [h] calculated from an active hydrogen equivalent weight of the epoxy resin curing agent [ep/h] is preferably in the range from 0.90 to 1.10, and more preferably 0.95 to 1.05. The closer this mixing ratio to an equivalent weight, the number of remaining functional groups decreases, thereby obtaining an epoxy resin with excellent performances.

[Epoxy resin cured product]

The epoxy resin composition according to the present disclosure can be cured by a known method, thereby obtaining an epoxy resin cured product. Curing conditions are suitably selected in accordance with the purpose of use and is not particularly limited. For example, the epoxy resin composition according to the present disclosure can be used at a common temperature condition of 15 to 30°C, and can also be heated to 30°C or more to heat curing. Further, the product obtained by curing the epoxy resin composition according to the present disclosure has advantages of achieving good drying property and water resistance, further with an outstanding appearance.

Examples

Hereinafter, the present invention will be described further in detail in reference to examples and comparative examples, but the present invention is not limited at all to these examples.

[Example 1] Synthesis of spiro bisamine

A 100 mL-SUS316 pressure resistant container (agitation type) was charged with 4.1 g of spiroglycol (SPG) (reagent manufactured by Tokyo Chemical Industry Co., Ltd.), 8.8 g of liquid ammonia (manufactured by MITSUBISHI GAS CHEMICAL COMPANY, INC.), and 0.41 g of 5% Ru/C catalyst (manufactured by N.E. CHEMCAT Corporation, type A) as the catalyst, and a hydrogen gas was introduced until a pressure in the system reached 4.1 MPa. The above catalyst was used after reduction at 250°C in a predetermined reduction device for 2 hours. Subsequently, inside the container was heated until the temperature reached 230°C, and the container was agitated while maintaining the same temperature, thereby allowing the amination reaction to proceed for 4.5 hours.

After completion of the reaction, the liquid ammonia was purged, the reaction product was dissolved in 200 g of methanol, and the catalyst in the liquid was removed by filtration and then analyzed using GC (manufactured by Shimadzu Corporation, type name "GC2010 PLUS", column: product name "HP-5ms", manufactured by Agilent Technologies Japan, Ltd., length 30 m × inner diameter 0.25 mm, film thickness 0.25 μm, conditions ··· carrier gas: He (constant pressure: 73.9 kPa), inlet temperature: 300°C, detector: FID, detector temperature: 325°C, column oven temperatures: started at 80°C, increased to 230°C at 20°C/min, increased to 240°C at 1°C /min, then increased to 325°C at 20°C/min, and maintained at 325°C for 20 minutes). The reaction yield was calculated by area percentage of the components detected by gas chromatography. As a result, the reaction yield of 2,2'-(2,4,8,10-tetraoxaspiro[5.5]undecane-3,9-diyl)bis(2-methylpropane-1-amine) represented by the above formula (2) was 97.6%.

[Example 2] Synthesis of spiro bisamine (scale up)

A 100 mL-SUS316 pressure resistant container (agitation type) was charged with 20.0 g of spiroglycol (reagent manufactured by Tokyo Chemical Industry Co., Ltd.), 10.1 g of liquid ammonia (manufactured by MITSUBISHI GAS CHEMICAL COMPANY, INC.), and 2.00 g of 5% Ru/C catalyst (manufactured by N.E. CHEMCAT Corporation, type A) as the catalyst, and a hydrogen gas was introduced until a pressure in the system reached 4.0 MPa. The above catalyst was used after reduction at 250°C in a predetermined reduction device for 2 hours. Subsequently, inside the container was heated until the temperature reached 228°C, and the container was agitated while maintaining the same temperature, thereby allowing the amination reaction to proceed for 4.5 hours.

After completion of the reaction, the liquid ammonia was purged, the reaction product was dissolved in 1000 g of methanol, and the catalyst in the liquid was removed by filtration and then analyzed using GC (manufactured by Shimadzu Corporation, type name "GC2010 PLUS", column: product name "HP-5ms", manufactured by Agilent Technologies Japan, Ltd., length 30 m × inner diameter 0.25 mm, film thickness 0.25 μm, conditions ··· carrier gas: He (constant pressure: 73.9 kPa), inlet temperature: 300°C, detector: FID, detector temperature: 325°C, column oven temperatures: started at 80°C, increased to 230°C at 20°C/min, increased to 240°C at 1°C /min, then increased to 325°C at 20°C/min, and maintained at 325°C for 20 minutes). The reaction yield was calculated by area percentage of the components detected by gas chromatography. As a result, the reaction yield of 2,2'-(2,4,8,10-tetraoxaspiro[5.5]undecane-3,9-diyl)bis(2-methylpropane-1-amine) represented by the above formula (2) was 88.1%.

[Example 3] Synthesis of dioxane bisamine

A 100 mL-SUS316 pressure resistant container (agitation type) was charged with 2.9 g of dioxane glycol, 8.9 g of liquid ammonia (manufactured by MITSUBISHI GAS CHEMICAL COMPANY, INC.), and 0.29 g of 5% Ru/C catalyst (manufactured by N.E. CHEMCAT Corporation, type A) as the catalyst, and a hydrogen gas was introduced until a pressure in the system reached 4.8 MPa. The above catalyst was used after reduction at 250°C in a predetermined reduction device for 2 hours. Subsequently, inside the container was heated until the temperature reached 232°C, and the container was agitated while maintaining the same temperature, thereby allowing the amination reaction to proceed for 4.5 hours.

After completion of the reaction, the liquid ammonia was purged, the reaction product was dissolved in 150 g of methanol, and the catalyst in the liquid was removed by filtration and then analyzed using GC (manufactured by Shimadzu Corporation, type name "GC2010 PLUS", column: product name "HP-5ms", manufactured by Agilent Technologies Japan, Ltd., length 30 m × inner diameter 0.25 mm, film thickness 0.25 μm, conditions ··· carrier gas: He (constant pressure: 73.9 kPa), inlet temperature: 300°C, detector: FID, detector temperature: 325°C, column oven temperatures: started at 80°C, increased to 230°C at 20°C/min, increased to 240°C at 1°C /min, then increased to 325°C at 20°C/min, and maintained at 325°C for 20 minutes). The reaction yield was calculated by area percentage of the components detected by gas chromatography. As a result, the reaction yield of 2-(5-(aminomethyl)-5-ethyl-1,3-dioxan-2-yl)-2-methylpropane-1-amine represented by the above formula (3) was 79.9%.

[Example 4]

An epoxy resin curing agent composition containing 48.2 mass% of the amino compound (spiro bisamine) obtained in Example 1, 11.8 mass% of an epoxy resin (manufactured by Mitsubishi Chemical Corporation, bisphenol A diglycidyl ether jER828), and 40 mass% of benzyl alcohol (manufactured by KANTO CHEMICAL CO., INC.) was prepared. Further, 4.2 g of the above curing agent composition was blended with 5.8 g of a base compound epoxy resin (manufactured by Mitsubishi Chemical Corporation, bisphenol A diglycidyl ether jER828) to prepare an epoxy resin composition. An active hydrogen equivalent weight (AHEW) of spiro bisamine calculated from the following formula (B), supposing that the purity of the isolated compound was 100% (the same hereinafter), was 174.

AHEW = (molecular weight calculated from identified chemical formula) ÷ (number of active hydrogens [number of functional groups of primary amine × 2 + number of functional groups of secondary amine]) ··· (B)

[Example 5]

An epoxy resin curing agent composition containing 44.6 mass% of the amino compound (dioxane bisamine) obtained in Example 3, 15.4 mass% of an epoxy resin (manufactured by Mitsubishi Chemical Corporation, bisphenol A diglycidyl ether jER828), and 40 mass% of benzyl alcohol (manufactured by KANTO CHEMICAL CO., INC.) was prepared. Further, 4.8 g of the above curing agent composition was blended with 5.2 g of a base compound epoxy resin (manufactured by Mitsubishi Chemical Corporation, bisphenol A diglycidyl ether jER828) to prepare an epoxy resin composition. An active hydrogen equivalent weight (AHEW) of dioxane bisamine calculated from the above formula (B), supposing that the purity of the isolated compound was 100%, was 135.

[Comparative Example 1]

An epoxy resin curing agent composition was prepared in the same manner as in Example 4, except that 39.4 mass% of 1,3-bis(aminomethyl)cyclohexane (1,3-BAC) (manufactured by MITSUBISHI GAS CHEMICAL COMPANY, INC.) and 20.6 mass% of an epoxy resin (manufactured by Mitsubishi Chemical Corporation, bisphenol A diglycidyl ether jER828) were used. Further, 3.5 g of the above curing agent composition was blended with 6.5 g of a base compound epoxy resin (manufactured by Mitsubishi Chemical Corporation, bisphenol A diglycidyl ether jER828) to prepare an epoxy resin composition. An active hydrogen equivalent weight (AHEW) of 1,3-BAC was 100.

[Comparative Example 2]

An epoxy resin curing agent composition was prepared in the same manner as in Example 4, except that 41.8 mass% of isophoronediamine (IPDA) (manufactured by Evonik Industries AG., Vestamine IPD) and 18.2 mass% of an epoxy resin (manufactured by Mitsubishi Chemical Corporation, bisphenol A diglycidyl ether jER828) were used. Further, 3.6 g of the above curing agent composition was blended with 6.4 g of a base compound epoxy resin (manufactured by Mitsubishi Chemical Corporation, bisphenol A diglycidyl ether jER828) to prepare an epoxy resin composition. An active hydrogen equivalent weight (AHEW) of IPDA was 106.

[Evaluation on characteristics]

<RCI curing rate>

The epoxy resin compositions obtained in Examples 4 and 5, and Comparative Examples 1 and 2 were applied onto a glass plate (manufactured by Taiyu Kizai Co., Ltd. 25 × 348 × 2.0 mm) as a base material under the conditions of 23°C, 50% R.H. using a 76 μm applicator to form coating films. The glass plate on which the coating film was formed was set on a paint dry time tester (manufactured by Taiyu Kizai Co., Ltd.), and streaks remained when needles of the tester scratched the coating film surface were observed to measure the time needed to reach each of the dry stages (Set to Touch, Dust Free, Dry through) by the following criteria. The shorter such a time is, the faster a curing rate (pot life is short) of the epoxy resin composition is.

Set to Touch: time at which needle marks start remaining on the glass plate
Dust Free: time at which needle marks float to the coating film surface from inside the coating film
Dry through: time at which no needle mark is remained on the coating film

<Coating film appearance>

The epoxy resin compositions obtained in Examples 4 and 5, and Comparative Examples 1 and 2 were applied onto a zinc phosphate treated iron plate (manufactured by PALTEK CORPORATION. SPCC-SD PB-N144 0.8 × 70 × 150 mm) as the base material under the conditions of 23°C, 50% R.H. using a 200 μm applicator to form coating films (thickness immediately after applied: 200 μm). These coating films were stored under the conditions of 23°C, 50% R.H., and the 1-day old appearance was visually observed to evaluate the transparency and gloss by the following criteria. Ex: Excellent, G: Good, F: Slightly Poor, P: Poor

<Coating film drying property>

The epoxy resin compositions obtained in Examples 4 and 5, and Comparative Examples 1 and 2 were applied onto a base material (zinc phosphate treated iron plate) by the same method as the above <Coating film appearance> to form coating films (thickness immediately after applied: 200 μm). These coating films were stored under the conditions of 23°C, 50% R.H., and the drying property of 1-, 2-, and 7-days old coating film was evaluated by touch by the following criteria.

Ex: Excellent (coating film is not sticky after being pressed by the thumb with a force of about 50 N and no fingerprint is remained)
G: Good (coating film is not sticky after being pressed by the thumb with a force of about 50 N, but a fingerprint is remained after touched)
F: Fair (coating film is sticky after being pressed by the thumb with a force of about 50 N)
P: Poor (coating film is sticky after being pressed by the thumb with a force of about 5 N)

<Pencil hardness>

The epoxy resin compositions obtained in Examples 4 and 5, and Comparative Examples 1 and 2 were applied onto a base material (zinc phosphate treated iron plate) by the same method as the above <Coating film appearance> to form coating films (thickness immediately after applied: 200 μm). These coating films were stored under the conditions of 23°C, 50% R.H., and the 7-days old pencil hardness was measured in conformity with JIS K5600-5-4: 1999.

<Water spot resistance>

The epoxy resin compositions obtained in Examples 4 and 5, and Comparative Examples 1 and 2 were applied onto a base material (zinc phosphate treated iron plate) by the same method as the above <Coating film appearance> to form coating films (thickness immediately after applied: 200 μm). These coating films were stored under the conditions of 23°C, 50% R.H., and 2 to 3 drops of pure water were put using a dropper on the surface of 1-, 2-, and 7-days old coating film, and the spots on which pure water was dropped were capped with 50 mL screw tube bottles. Water was wiped off 24 hours later, and the appearance was visually observed and evaluated by the following criteria.
Ex: No change, G: Slightly changed but good, F: Changes were observed

<Chemical resistance>

The epoxy resin compositions obtained in Examples 4 and 5, and Comparative Examples 1 and 2 were applied onto a base material (zinc phosphate treated iron plate) by the same method as the above <Coating film appearance> to form coating films (thickness immediately after applied: 200 μm). These coating films were cured for 7 days under the conditions of 23°C, 50% R.H., and 2 to 3 drops of pure water, or 5% brine, were put using a dropper on the coating film surface, and the spots on which pure water or 5% brine was dropped were capped with 50 mL screw tube bottles. Water or the brine was wiped off 1, 2, 3, and 4 weeks later, and the appearance was visually observed and evaluated by the following criteria.

Ex: No change
G: Rough surface
F: Surface was whitened, and a few rust spots were confirmed
P: Rough surface, and rust spots were confirmed overall

Table 1 collectively shows the evaluation results of each of the above characteristics. As evident from Table 1, it was confirmed that the epoxy resin compositions of Examples significantly excel in a longer pot life, water spot resistance, and chemical resistance compared with the epoxy resin compositions of Comparative Examples in which the conventional epoxy resin curing agents was used. Further, it is presumed that the amino compounds according to the present disclosure other than Examples can also similarly provide equivalent effects by the same mechanism as Examples, because such compounds have a quaternary carbon at the β-position of an amino group, and have the A moiety containing an -O- bond bonded to the carbon at the γ-position of the amino group.

[Table 1]

| Curing conditions: 23°C/50% RH (Epoxy resin curing agent) | | Example 4 (Spiro bisamine) | Example 5 (Dioxane bisamine) | Comparative Example 1 (1,3-BAC) | Comparative Example 2 (IPDA) |
|---|---|---|---|---|---|
| Active hydrogen equivalent weight (AHEW) | | 174 | 135 | 100 | 106 |
| RCI curing rate (hr: min) | Set to Touch | 2:45 | 2:30 | 1:00 | - |
| | Dust Free | 6:15 | 5:15 | 2:15 | 4:15 |
| | Dry through | 18:45 | 19:30 | 3:30 | - |
| Coating film appearance (transparency/gloss) | | Ex/Ex | Ex/Ex | Ex/Ex | Ex/Ex |
| Coating film dry property (1/2/7 days old) | | Ex/Ex/Ex | Ex/Ex/Ex | Ex/Ex/Ex | Ex/Ex/Ex |
| Pencil hardness (7 days old) | | H | H | H | H |
| Water spot resistance (1/2/7 days old) | | Ex/Ex/Ex | G/Ex/Ex | F/F/F | F/F/F |
| Chemical resistance (1/2/3/4 weeks old) | 5% Brine | Ex/Ex/Ex/G | Ex/Ex/Ex/Ex | F (Rough surface, rust spots)/F/F/F | F (Whitened) /F/F/F |
| | Water | Ex/Ex/Ex/Ex | Ex/Ex/Ex/Ex | G (Rough surface)/F (Whitened)/F/F | P (Rust spots, whitened)/P/P/P |

As described above, the amino compounds and the method for producing the same according to the present disclosure, and the epoxy resin curing agent, the epoxy resin composition, and the epoxy resin cured product using the same have good coating film appearance and coating film drying property and excel particularly in a longer pot life, water spot resistance, and chemical resistance compared with the conventional products, and thus can be widely and effectively used in various purposes of use where these performances are demanded. The present application is based on the Japanese Patent Application No. 2021-091643 filed on May 31, 2021, and its contents are incorporated herein.

## Claims

1. An amino compound having a quaternary carbon at the β-position of an amino group and represented by the following formula (1).

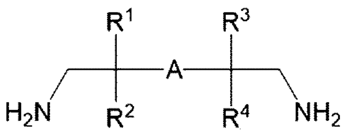

···(1)

wherein,

A is any of the following (α), (β), and (γ);

(α) a hydrocarbon group independently having at least one -O- bond bonded to the carbon at the γ-position of the amino group, and having 2 to 30 carbon atoms and optionally having a substituent,
(β) a hydrocarbon group having at least one -O-bond bonded to the carbon at the γ-position of the amino group while bonding to any one of $R^1$, $R^2$, $R^3$, and $R^4$, and having 2 to 30 carbon atoms and optionally having a substituent,
(γ) a hydrocarbon group having at least one -O-bond bonded to the carbon at the γ-position of the amino group while bonding to $R^1$ or $R^2$ and $R^3$ or $R^4$, and having 2 to 30 carbon atoms and optionally having a substituent, wherein

$R^1$, $R^2$, $R^3$, and $R^4$ are each independently a hydrocarbon group having 1 to 10 carbon atoms and optionally having a substituent (provided that a hydrocarbon group bonded to A is excluded).

2. The amino compound according to claim 1,

wherein A independently has a dioxane structure, or
A has a dioxane structure while bonding to any one of $R^1$, $R^2$, $R^3$, and $R^4$, or
A has a dioxane structure while bonding to $R^1$ or $R^2$ and $R^3$ or $R^4$.

3. The amino compound according to claim 1 or 2, represented by the following formula (2) or formula (3).

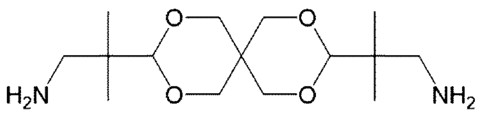

···(2)

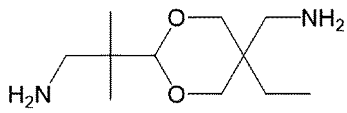

···(3)

4. A method for producing an amino compound comprising:

a step of aminizing a polyol compound represented by the following formula (1a) in the presence of a catalyst and ammonia to obtain an amino compound having a quaternary carbon at the β-position of an amino group and represented by the following formula (1).

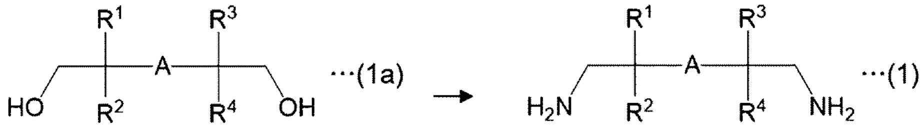
···(1a) ···(1)

In formula (1) and formula (1a),
A is any of the following (α), (β), and (γ);

(α) a hydrocarbon group independently having at least one -O- bond bonded to the carbon at the γ-position of the amino group, and having 2 to 30 carbon atoms and optionally having a substituent,
(β) a hydrocarbon group having at least one -O-bond bonded to the carbon at the γ-position of the amino group while bonding to any one of $R^1$, $R^2$, $R^3$, and $R^4$, and having 2 to 30 carbon atoms and optionally having a substituent,
(γ) a hydrocarbon group having at least one -O-bond bonded to the carbon at the γ-position of the amino group while bonding to $R^1$ or $R^2$ and $R^3$ or $R^4$, and having 2 to 30 carbon atoms and optionally having a substituent, wherein

$R^1$, $R^2$, $R^3$, and $R^4$ are each independently a hydrocarbon group having 1 to 10 carbon atoms and optionally having a substituent (provided that a hydrocarbon group bonded to A is excluded).

**5.** The method for producing the amino compound according to claim 4,

wherein A independently has a dioxane structure, or
A has a dioxane structure while bonding to any one of $R^1$, $R^2$, $R^3$, and $R^4$, or
A has a dioxane structure while bonding to $R^1$ or $R^2$ and $R^3$ or $R^4$.

**6.** A method for producing an amino compound comprising:

a step of aminizing a polyol compound represented by the following formula (2a) in the presence of a catalyst and ammonia to obtain an amino compound represented by the following formula (2), or
a step of aminizing a polyol compound represented by the following formula (3a) in the presence of a catalyst and ammonia to obtain an amino compound represented by the following formula (3).

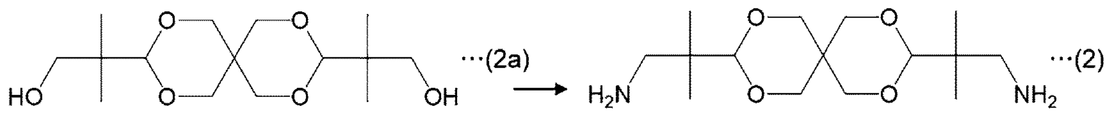
···(2a) ···(2)

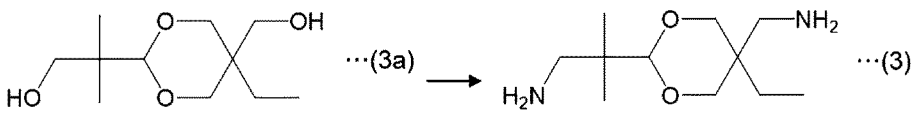
···(3a) ···(3)

**7.** An epoxy resin curing agent, comprising the amino compound according to any one of claims 1 to 3.

**8.** An epoxy resin composition comprising the epoxy resin curing agent according to claim 7, and an epoxy resin.

**9.** An epoxy resin cured product obtained by curing the epoxy resin composition according to claim 8.

## INTERNATIONAL SEARCH REPORT

International application No. **PCT/JP2022/019776**

**A. CLASSIFICATION OF SUBJECT MATTER**

***C07D 319/06***(2006.01)i; ***C08G 59/50***(2006.01)i; ***C07B 61/00***(2006.01)i; ***C08L 63/00***(2006.01)i; ***C07D 493/10***(2006.01)i
FI: C07D319/06; C08G59/50; C08L63/00 C; C07B61/00 300; C07D493/10 D CSP

According to International Patent Classification (IPC) or to both national classification and IPC

**B. FIELDS SEARCHED**

Minimum documentation searched (classification system followed by classification symbols)

C07D319/06; C08G59/50; C07B61/00; C08L63/00; C07D493/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 62-169776 A (TORAY IND INC) 25 July 1987 (1987-07-25)<br>claims, examples | 1-2<br>3-9 |
| X<br>A | CN 102786544 A (CHANGCHUN INSTITUTE OF APPLIED CHEMISTRY, CHINISE ACADEMY OF SCIENCE) 21 November 2012 (2012-11-21)<br>claim 5, examples | 1-2<br>3-9 |
| X<br>A | QU, Zhi et al. Living and stereoselective polymerization of rac-lactide by bimetallic aluminum schiff-base complexes. Journal of Polymer Science, Part A: Polymer Chemistry. 2014, 52, 1344-1352<br>compound 3 | 1-2<br>3-9 |
| X<br>A | JP 2014-224194 A (NISSAN CHEMICAL IND LTD) 04 December 2014 (2014-12-04)<br>paragraph [0016], compound 4 | 1-2<br>3-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2022** | **19 July 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No. PCT/JP2022/019776

C. DOCUMENTS CONSIDERED TO BE RELEVANT

| Category* | Citation of document, with indication, where appropriate, of the relevant passages | Relevant to claim No. |
|---|---|---|
| X<br>A | JP 2014-118442 A (FUJIFILM CORP) 30 June 2014 (2014-06-30)<br>paragraph [0064], multivalent amines 6, 9 | 1<br>2-9 |
| X<br>A | BALIJA, Amy M. et al. Substituted 1,3,5-Triazaadamantanes: Biocompatible and degradable building blocks. Angew.Chem.Int.Ed. 2008, 47, 8072-8074, Supp.Info.<br>compound 6 | 1<br>2-9 |
| X<br>A | KEANA, John F. W. et al. Novel nitroxids for spin-labelling, -trapping, and magnetic resonance imaging applications. Pure & Appl. Chem. 1990, 62(2), 201-205<br>hexaamine 35a | 1<br>2-9 |
| X<br>A | DORNOW, Alfred et al. Uber die Reduktion substituierter Cyanessigsaure-athylester (Reduktionen mit LiALH4 VIII. Mitteil.). Chem. Ber. 1954, 87(7), 985-990<br>p. 988, 1-Amino-2.2-dibenzyl-propanol, Oxalat | 1<br>2-9 |
| X<br>A | JOO, Young-Hyuk et al. Polynitramino compounds outperform PETN. Chem. Comm. 2010, 46, 142-144<br>polynitramines 6, 7 | 1<br>2-9 |
| X<br>A | CN 102872700 A (NANJING UNIVERSITY OF INFORMATION SCIENCE & TECHNOLOGY) 16 January 2013 (2013-01-16)<br>compound II-5 | 1<br>2-9 |
| X<br>A | JP 2005-538050 A (BIOMIRA, INC.) 15 December 2005 (2005-12-15)<br>fig. 11, compound 28 | 1<br>2-9 |
| A | JP 2018-515610 A (NEWBIO THERAPEUTICS, INC.) 14 June 2018 (2018-06-14)<br>example 2, compound 17 | 1-9 |
| A | JP 9-202821 A (MITSUBISHI GAS CHEM CO INC) 05 August 1997 (1997-08-05)<br>claims, table 1 | 1-9 |
| A | JP 2020-172634 A (MITSUBISHI GAS CHEM CO INC) 22 October 2020 (2020-10-22)<br>claims, tables 1-4 | 1-9 |
| A | JP 2019-89971 A (DAINIPPON INK & CHEMICALS) 13 June 2019 (2019-06-13)<br>claims, examples | 1-9 |

Form PCT/ISA/210 (second sheet) (January 2015)

**INTERNATIONAL SEARCH REPORT**
**Information on patent family members**

International application No.
**PCT/JP2022/019776**

| Patent document cited in search report | Publication date (day/month/year) | Patent family member(s) | Publication date (day/month/year) |
|---|---|---|---|
| JP 62-169776 A | 25 July 1987 | (Family: none) | |
| CN 102786544 A | 21 November 2012 | (Family: none) | |
| JP 2014-224194 A | 04 December 2014 | (Family: none) | |
| JP 2014-118442 A | 30 June 2014 | (Family: none) | |
| CN 102872700 A | 16 January 2013 | (Family: none) | |
| JP 2005-538050 A | 15 December 2005 | US 2006/0040891 A1<br>fig. 11, compound 28<br>WO 2003/094850 A2<br>CA 2485253 A<br>EP 1549322 A1<br>US 2012/0003295 A1<br>AU 2003228966 A1 | |
| JP 2018-515610 A | 14 June 2018 | US 2018/0147295 A1<br>example 2, compound 17<br>WO 2016/192528 A1<br>EP 3302570 A1<br>CN 106267225 A | |
| JP 9-202821 A | 05 August 1997 | (Family: none) | |
| JP 2020-172634 A | 22 October 2020 | (Family: none) | |
| JP 2019-89971 A | 13 June 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021091643 A